(19) Europäisches Patentamt
European Patent Office
Office européen des brevets

(11) Veröffentlichungsnummer : **0 025 931**
**B1**

# (12) EUROPÄISCHE PATENTSCHRIFT

(45) Veröffentlichungstag der Patentschrift : **09.03.83**

(51) Int. Cl.³ : **C 07 D307/32**, C 07 D307/30

(21) Anmeldenummer : **80105374.5**

(22) Anmeldetag : **09.09.80**

## (54) Verfahren zur Herstellung von 5-(2,2,-Dihalogenvinyl)-4,4-dialkyl-tetrahydrofuran-2-onen.

(30) Priorität : **19.09.79 DE 2937763**

(43) Veröffentlichungstag der Anmeldung : **01.04.81 Patentblatt 81/13**

(45) Bekanntmachung des Hinweises auf die Patenterteilung : **09.03.83 Patentblatt 83/10**

(84) Benannte Vertragsstaaten : **AT BE CH DE FR GB IT LI NL SE**

(56) Entgegenhaltungen :
**DE A 2 630 981**
**DE A 2 813 336**

**PATENT ABSTRACTS OF JAPAN, unexamined applications, Sektion C, Band 1, Nr. 131, 28. Oktober 1977, The Patent Office Japanese Government, Seite 2880 C 77.**

**PATENT ABSTRACTS OF JAPAN, unexamined applications, Sektion C, Band 1, Nr. 131, 28. Oktober 1977, The Patent Office Japanese Government, Seite 2881 C 77.**

**PATENT ABSTRACTS OF JAPAN, unexamined applications, Sektion C, Band 1, Nr. 131, 28. Oktober 1977, The Patent Office Japanese Government, Seite 2927 C 77.**

(73) Patentinhaber : **BASF Aktiengesellschaft**
**Carl-Bosch-Strasse 38**
**D-6700 Ludwigshafen (DE)**

(72) Erfinder : **Kropp, Rudolf**
**Sprottauer Strasse 2**
**D-6703 Limburgerhof (DE)**
Erfinder : **Fischer, Martin, Dr.**
**Elbingerweg 1**
**D-6700 Ludwigshafen (DE)**
Erfinder : **Halbritter, Klaus, Dr.**
**Schwarzwaldstrasse 19**
**D-6800 Mannheim 1 (DE)**

(56) Entgegenhaltungen :
**PATENT ABSTRACTS OF JAPAN, unexamined applications, Sektion C, Band 1, Nr. 131, 28. Oktober 1977 The Patent Office Japanese Government, Seite 3469 C 77.**

**Die Akte enthält technische Angaben, die nach dem Eingang der Anmeldung eingereicht wurden und die nicht in dieser Patentschrift enthalten sind.**

**EP 0 025 931 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von 5-(2,2-Dihalogenvinyl)-4,4-dialkyl-tetrahydrofuran-2-onen

Die Erfindung betrifft ein Verfahren zur Herstellung von 5-(2,2-Dihalogenvinyl)-4,4-dialkyl-tetrahydrofuran-2-onen durch Umsetzung von N,N-disubstituierten Carbonsäureamiden mit Tetrahalogenmethanen und anschließende Hydrolyse der so erhaltenen Iminiumsalze.

Es sind bereits mehrere Verfahren zur Herstellung von 5-(2,2-Dihalogenvinyl)-4,4-dialkyl-tetrahydrofuran-2-onen bekannt. So wird in der DE-OS 26 21 831 die Umsetzung von 4,6,6,6-Tetrahalogen-3,3-dimethyl-hexansäure mit Alkalialkoholat zu 5-(2,2-Dihalogenvinyl)-4,4-dimethyl-tetrahydrofuran-2-on beschrieben. Diese Lactone können auch durch Behandlung von 4,6,6,6-Tetrahalogen-3,3-dimethylhexancarbonsäureäthylester mit Natriumhydroxid (DE-OS 26 24 351), durch Kochen mit Wasser bzw. Erhitzen auf 180 bis 200 °C aus 4,6,6-Trihalogen-3,3-dimethyl-hex-5-en-säuremethylester (JP-OS 77/83 457, JP-OS 77/83 459) oder durch Behandlung von 4,3,2,2-Tetrahalogen-5,5-dimethyl-cyclohexanon mit Alkalihydroxid (DE-OS 26 23 777) erhalten werden. Ein anderer Syntheseweg führt über die Addition von Essigsäure an 1,1-Dichlor-4-methyl-1,3-pentadien in Gegenwart von Mangan(III)-acetat (JP-OS 77/57 163) zum entsprechenden Dichlorvinyl-substituierten Lacton. Von Nachteil bei diesen Verfahren sind die zum Teil geringe Ausbeute, umständliche Verfahrensweisen und die Verwendung schwer zugänglicher Ausgangsstoffe.

Es wurde nun gefunden, daß man 5-(2,2-Dihalogenvinyl)-4,4-dialkyl-tetrahydrofuran-2-one der Formel I

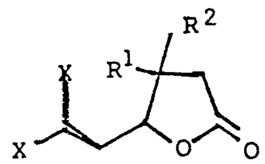

(I)

in der $R^1$ und $R^2$ je einen Alkylrest mit 1 bis 4 Kohlenstoffatomen und X Halogen bedeuten, in vorteilhafter Weise erhält, wenn man ein Carbonsäureamid der Formel II

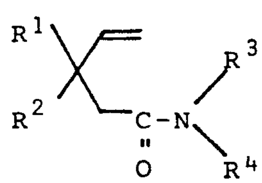

(II)

in der $R^1$ und $R^2$ je einen Alkylrest mit 1 bis 4 Kohlenstoffatomen und $R^3$ und $R^4$ je einen Alkylrest mit 1 bis 4 Kohlenstoffatomen, einen Aralkylrest mit 7 bis 9 Kohlenstoffatomen oder einen Arylrest mit 6 bis 10 Kohlenstoffatomen bedeuten oder zusammen mit dem Stickstoffatom, dessen Substituenten sie sind, einen 5- oder 6-gliedrigen gesättigten Ring bilden, der noch ein weiteres Heteroatom enthalten kann, mit einem Tetrahalogenmethan der Formel III

$$CX_4 \qquad \text{(III)}$$

in der X Halogen bedeutet, in Gegenwart eines Initiators und eines organischen Verdünnungs- oder Lösungsmittels bei einer Temperatur im Bereich zwischen 120 und 160 °C zu einem Iminiumsalz der Formel IV

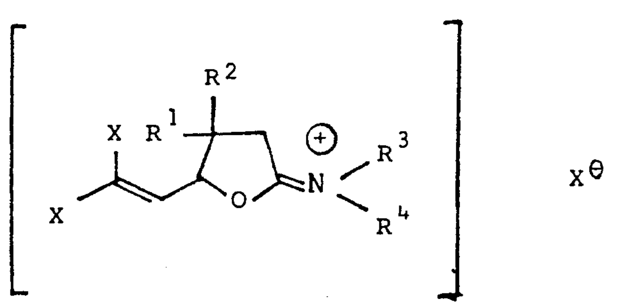

(IV)

in der $R^1$, $R^2$, $R^3$, $R^4$ und X die obengenannten Bedeutungen haben, umsetzt und dieses Iminiumsalz anschließend hydrolysiert.

Nach dem erfindungsgemäßen Verfahren lassen sich 5-(2,2-Dihalogenvinyl)-4,4-dialkyl-tetrahydrofuran-2-one herstellen, die als Zwischenprodukte für die Synthese insektizider Wirkstoffe dienen. Die Verbindungen lassen sich durch Behandeln mit einem anorganischen Säurehalogenid, beispielsweise

einem Thionylhalogenid, und anschließender Umsetzung mit einer Base in 2-(2,2-Dihalogenvinyl)-3,3-dialkyl-cyclopropancarbonsäureester, beispielsweise in 2-(2,2-Dihalogenvinyl)-3,3-dimethyl-cyclopropancarbonsäure-alkylester, überführen (DE-OS 26 21 831), die Ausgangsprodukte für die Synthese insektizider Wirkstoffe aus der Klasse der Pyrethroide sind. Die Synthese der 5-(2,2-Dihalogenvinyl)-4,4-dialkyl-tetrahydrofuran-2-one nach dem erfindungsgemäßen Verfahren ist besonders vorteilhaft und wirtschaftlich, da sie drei Grundvoraussetzungen für die technische Nutzung, nämlich gut zugängliche Ausgangsprodukte, einfache Durchführung und hohe Ausbeute, erfüllt.

Die als Ausgangsstoffe verwendeten Carbonsäureamide der Formel II sind bekannt und können in Anlehnung an bekannte Verfahren hergestellt werden (JP-OS 77/83 411 ; DE-OS 27 32 213). Die Carbonsäureamide können außerdem durch Umsetzung von Acetamidacetalen oder Ketenacetalaminalen mit 3-Methyl-but-2-en-1-ol erhalten werden.

Bevorzugte Ausgangsstoffe der Formel II sind 3,3-Dimethyl-pent-4-ensäure-N,N-dimethylamid, 3,3-Diäthyl-pent-4-ensäure-N,N-dimethylamid, 3-Methyl-3-n-propyl-pent-4-ensäure-N,N-dimethylamid, 3,3-Dimethyl-pent-4-ensäure-N-methyl-N-phenylamid, 3,3-Dimethyl-pent-4-ensäure-N-methyl-N-benzylamid, 3,3-Dimethyl-pent-4-ensäure-pyrrolidid und 3,3-Dimethyl-pent-4-ensäure-morpholid.

Für die Umsetzung geeignete Tetrahalogenmethane der Formel III sind beispielsweise Tetrachlormethan, Tetrabrommethan und Bromtrichlormethan.

Die Ausgangsstoffe der Formeln II und III können in stöchiometrischer Menge umgesetzt werden. Es ist jedoch von Vorteil, den Ausgangsstoff der Formel III im Überschuß einzusetzen, d. h. im allgemeinen in einer Menge von 1 bis 20 Mol, vorteilhaft 3 bis 10 Mol, pro Mol Ausgangsstoff der Formel II.

Als Initiatoren für die Umsetzung der Carbonsäureamide mit den Tetrahalogenmethanen zu Iminiumsalzen eignen sich organische Peroxide oder Perester, wie Di-tert.-butylperoxid, Di-benzoylperoxid oder 2-Äthyl-hexansäure-tert.-butylperester, Azo-bis-isobuttersäurenitril, Redoxsysteme, die Eisen- und/oder Kupferionen, wie $Fe^{3+}/Fe^{2+}$, $Cu^{2+}/Cu^{1+}$, $Cu^{2+}/Fe^{2+}$, enthalten sowie UV-Licht. Pro Mol Carbonsäureamid der Formel II werden 0,01 bis 0,5 Mol, vorzugsweise 0,05 bis 0,2 Mol, Initiator zugesetzt.

Als organische Verdünnungs- oder Lösungsmittel kommen ggf. chlorierte aliphatische und aromatische Kohlenwasserstoffe, wie Heptan, Cyclohexan, Benzol, Toluol oder Chlorbenzol, in Betracht. Zweckmäßigerweise verwendet man jedoch einen Überschuß an Tetrahalogenmethan der Formel III als Lösungsmittel. Die Menge an Lösungsmittel pro Verbindung der Formel II kann in weiten Bereichen variiert werden ; sie kann 100 bis 2 000 Gew.%, vorteilhafterweise 500 bis 1 000 Gew.%, bezogen auf Carbonsäureamid, betragen.

Die Umsetzung der Carbonsäureamide der Formel II mit Tetrahalogenmethanen der Formel III zu Iminiumsalzen der Formel IV wird bei einer Temperatur im Bereich von 120 bis 160 °C, vorzugsweise 130 bis 150 °C, drucklos oder unter Druck, diskontinuierlich oder kontinuierlich, durchgeführt. Arbeitet man drucklos mit Reaktionsteilnehmern und/oder Lösungsmitteln, deren Siedepunkte unter 120 °C liegen, so heizt man das Reaktionsgemisch unter gleichzeitigem Abdestillieren der überschüssigen Reaktionspartner, ausgehend von Zimmertemperatur, innerhalb von 1 bis 20 Stunden auf die Reaktionstemperatur auf. Führt man die Reaktion in Gegenwart von Peroxiden oder Perestern, deren Zerfallstemperaturen oberhalb 70 °C liegen, aus, so empfiehlt es sich, das Reaktionsgemisch zunächst 1 bis 15 Stunden lang auf eine Temperatur von mindestens 70 °C und dann auf eine Temperatur im Bereich von 120 bis 160 °C zu erhitzen. Bei Verwendung von UV-Licht als Initiator genügt es, die Temperaturen zunächst für 10 bis 20 Stunden nur bei 20 °C zu halten und dann auf 120 bis 160 °C zu steigern.

Die durch Umsetzung der Carbonsäureamide der Formel II mit Tetrahalogenmethanen der Formel III erhaltenen Iminiumsalze der Formel IV sind neu. In dieser Formel bedeutet X Halogen, vorzugsweise Chlor oder Brom, wobei sowohl die Halogensubstituenten der Vinylgruppe als auch das Anion verschieden sein können. $R^1$ und $R^2$ in Formel IV bedeuten je einen Alkylrest mit 1 bis 4 Kohlenstoffatomen, insbesondere Methyl. $R^3$ und $R^4$ können für einen Alkylrest mit 1 bis 4 Kohlenstoffatomen, insbesondere Methyl, Äthyl, Isopropyl, einen Aralkylrest mit 7 bis 9 Kohlenstoffatomen, insbesondere Benzyl, oder einen Arylrest mit 6 bis 10 Kohlenstoffatomen, insbesondere Phenyl, stehen. $R^3$ und $R^4$ können auch zusammen mit dem Stickstoffatom, dessen Substituenten sie sind, einen 5- oder 6-gliedrigen Ring bilden, der noch ein weiteres Heteroatom, insbesondere Sauerstoff, enthalten kann. Beispiele für solche Ringe sind Pyrrolidin, Piperidin und Morpholin.

Die Iminiumsalze der Formel IV lassen sich durch Behandeln mit der 1- bis 20-fachen, vorzugsweise 3- bis 5-fachen, Gewichtsmenge Wasser bei einer Temperatur im Bereich zwischen 20 und 150 °C, vorzugsweise zwischen 80 und 100 °C, in die Verbindungen der Formel I überführen, wobei die Umsetzung drucklos oder unter Druck erfolgen kann. Diese Hydrolyse kann in Gegenwart des organischen Verdünnungs- oder Lösungsmittels durchgeführt werden, in dessen Anwesenheit die Synthese des Iminiumsalzes durchgeführt wurde. Die dann anfallenden Tetrahydrofuranone der Formel I können in üblicher Weise durch Abtrennung von der wäßrigen Phase oder durch Extraktion aus dem wäßrigen Reaktionsgemisch isoliert und durch Destillation oder Kristallisation gereinigt werden.

Das erfindungsgemäße Verfahren wird durch die folgenden Beispiele erläutert.

## Beispiel 1

Zu 3 080 g (20 Mol) Tetrachlormethan gibt man bei 70 bis 75 °C unter Rühren 310 g (2 Mol) 3,3-Dimethyl-pent-4-ensäure-N,N-dimethylamid und 46 g (0,2 Mol) 2-Ethylhexansäure-tert.-butylperester kontinuierlich innerhalb von 3 Stunden zu. Anschließend rührt man noch 2 Stunden bei 75 bis 80 °C und destilliert dann bis zu einer Innentemperatur von 140 °C 2 661 g (17,26 Mol) Tetrachlormethan ab.

Man rührt noch weitere 4 Stunden bei 140 °C, versetzt mit 1 500 g Wasser und rührt das Gemisch 1 Stunde lang bei 90 °C. Nach dem Abkühlen wird die untere Phase des Reaktionsgemisches abgetrennt und destilliert. Man erhält 351 g (1,68 Mol) 5-(2,2-Dichlorvinyl)-4,4-dimethyltetrahydrofuran-2-on vom Sdp. 101 °C/0,27 mbar, entsprechend einer Ausbeute von 84 % der Theorie. $C_8H_{10}O_2Cl_2$ (209)

Ber.: C 45,96 %; H 4,82 %; Cl 33,91 %;
Gef.: C 46,3 %; H 4,9 %; Cl 34,5 %;
NMR (220 MHz): 3 H 1,05 ppm (s)
3 H 1,22 ppm (s)
2 H 2,45 ppm (dd)
1 H 4,95 ppm (d)
1 H 5,98 ppm (d)
$N_D^{20}$: 1,498 9

## Beispiel 2

Eine Mischung von 40 g (0,26 Mol) 3,3-Dimethyl-pent-4-ensäure-N,N-dimethylamid, 231 g (1,5 Mol) Tetrachlormethan und 7 g (0,05 Mol) Di-tert.-butylperoxid wird in einem Autoklaven aus rostfreiem Stahl 6 Stunden bei 140 °C gerührt. Aus dem Reaktionsgemisch werden dann bei vermindertem Druck 180 g (1,17 Mol) Tetrachlormethan abdestilliert und der Rückstand mit 200 g Wasser 1 Stunde bei 80 bis 90 °C gerührt. Durch Extraktion mit Methylenchlorid und anschließende Destillation der Methylenchloridlösung erhält man 39 g (0,19 Mol) 5-(2,2-Dichlorvinyl)-4,4-dimethyl-tetrahydrofuran-2-on vom Sdp. 101 °C/0,27 mbar.

## Beispiel 3

Eine Lösung von 132 g (0,4 Mol) Tetrabrommethan in 250 ml Chlorbenzol wird bei 130 °C gerührt. Innerhalb 1 Stunde tropft man eine Mischung von 31 g (0,2 Mol) 3,3-Dimethyl-pent-4-ensäure-N,N-dimethylamid und 3 g (0,02 Mol) Ditert.-butylperoxid ein und rührt noch 1 Stunde bei 130 °C. Das abgekühlte Reaktionsgemisch wird mit 300 ml Wasser gerührt, die wäßrige Lösung abgetrennt und destilliert. Man erhält 80 g (0,24 Mol) Tetrabrommethan zurück.

Die abgetrennte wäßrige Lösung wird 2 Stunden bei 80 bis 90 °C gerührt und nach dem Abkühlen mit Methylenchlorid extrahiert. Nach dem Abdestillieren des Methylenchlorids erhält man 30 g (0,1 Mol) 5-(2,2-Dibromvinyl)-4,4-dimethyl-tetrahydrofuran-2-on vom Schmp. 73 bis 74 °C.

$C_8H_{10}Br_2$ (298)
Ber.: C 32,25 %; H 3,38 %; Br 53,63 %;
Gef.: C 32,8 %; H 3,4 %; Br 52,9 %;
NMR (100 MHz): 3 H 1,08 ppm (s)
3 H 1,23 ppm (s)
2 H 2,4 ppm (d)
1 H 4,75 ppm (d)
1 H 6,45 ppm (d)

## Beispiel 4

124 g (0,8 Mol) 3,3-Dimethyl-pent-4-en-säure-N,N-dimethylamid, 1 600 g (10,4 Mol) Tetrachlormethan und 8 g Benzoylperoxid werden 7 Stunden bei 75 bis 80 °C gerührt. Nach Zugabe weiterer 8 g Benzoylperoxid wird weitere 7 Stunden auf 75 bis 80 °C erwärmt. Bei dem anschließenden Erhitzen bis zu einer Badtemperatur von 150 °C erhält man neben dem überschüssigen Tetrachlormethan 51 g 3,3-Dimethyl-pent-4-ensäure-N,N-dimethylamid zurück. Nach dem Erkalten des Rückstandes kristallisiert das N,N-Dimethyl-[5-(2,2-dichlorvinyl)-4,4-dimethyl-tetrahydrofuryl-2]-iminiumchlorid als hygroskopische Substanz vom Schmp. 73 bis 78 °C aus.

$C_{10}H_{16}ONCl_3$ (272,5)
Ber.: C 44,0 %; H 5,87 %; N 5,14 %; Cl 39,1 %;
Gef.: C 44,7 %; H 6,4 %; N 5,1 %; Cl 35,1 %.

Das Chlorid wird in 250 ml Wasser 3 Stunden lang bei 90 °C gerührt. Nach dem Abtrennen und Destillieren der organischen Phase erhält man 84 g (0,4 Mol) 5-(2,2-Dichlorvinyl)-4,4-dimethyl-tetrahydrofuran-2-on.

## Beispiel 5

In eine Lösung von 1 g Azo-bis-isobuttersäurenitril in 308 g (2 Mol) Tetrachlormethan tropft man bei 75 bis 80 °C innerhalb 3 Stunden 31 g (0,2 Mol) 3,3-Dimethyl-pent-4-ensäure-N,N-dimethylamid. Man

rührt 1,5 Stunden bei 75 bis 80 °C, gibt dann nochmals 1 g Azo-bis-isobuttersäurenitril zu und hält die Temperatur weitere 2 Stunden bei 75 bis 80 °C. Nach dem Abdestillieren der nicht umgesetzten Ausgangsprodukte bei einer Badtemperatur von maximal 150 °C verbleibt ein Rückstand, der mit 100 ml Wasser 1 Stunde lang bei 80 bis 90 °C gerührt wird. Dabei erhält man als wasserunlösliche Phase 17 g (0,08 Mol) 5-(2,2-Dichlorvinyl)-4,4-dimethyl-tetrahydrofuran-2-on.

Beispiel 6

Eine Lösung von 31 g (0,2 Mol) 3,3-Dimethyl-pent-4-ensäure-N,N-dimethylamid in 436 g (2,83 Mol) Tetrachlormethan wird 15 Stunden bei 20 °C mit einer 300 W-Quecksilber-Hochdrucklampe bestrahlt. Bei der Destillation der Reaktionslösung erhält man neben dem überschüssigem Tetrachlormethan 19 g 3,3-Dimethyl-pent-4-ensäure-N,N-dimethylamid zurück sowie bei 0,013 mbar und 130 bis 137 °C 10 g N,N-Dimethyl-[5-(2,2-dichlorvinyl)-4,4-dimethyl-tetrahydrofuryl-2]-iminiumchlorid.

Zur Identifizierung des Salzes wird durch Auflösen des Chlorids in Wasser und Zugabe von Perchlorsäure das Perchlorat ausgefällt. Es schmilzt bei 158 bis 160 °C und hat folgende Analysenwerte :

$C_{10}H_{16}O_5NCl_3$ (336,5)

Ber. : N 4,16 % ; Cl 31,65 % ;

Gef. : N 3,9 % ; Cl 31,1 %

NMR (270 MHz) 3 H 1,22 ppm (s)
3 H 1,35 ppm (s)
2 H 3,3 ppm (dd)
3 H 3,38 ppm (s)
3 H 3,42 ppm (s)
1 H 5,55 ppm (d)
1 H 6,15 ppm (d)

Aus 9 g Chlorid, die man eine Stunde lang in 50 ml Wasser auf 90 °C erwärmt, erhält man 5 g 5-(2,2-Dichlorvinyl)-4,4-dimethyl-tetrahydrofuran-2-on als wasserunlösliche Phase.

## Anspruch

Verfahren zur Herstellung von 5-(2,2-Dihalogenvinyl)-4,4-dialkyl-tetrahydro-furan-2-onen der Formel I

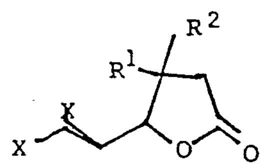

(I)

in der $R^1$ und $R^2$ je einen Alkylrest mit 1 bis 4 Kohlenstoffatomen und X Halogen bedeuten, dadurch gekennzeichnet, daß man ein Carbonsäureamid der Formel II

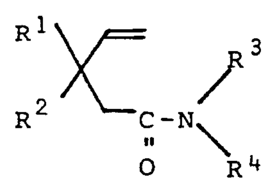

(II)

in der $R^1$ und $R^2$ je einen Alkylrest mit 1 bis 4 Kohlenstoffatomen und $R^3$ und $R^4$ je einen Alkylrest mit 1 bis 4 Kohlenstoffatomen, einen Aralkylrest mit 7 bis 9 Kohlenstoffatomen oder einen Arylrest mit 6 bis 10 Kohlenstoffatomen bedeuten oder zusammen mit dem Stickstoffatom, dessen Substituenten sie sind, einen 5- oder 6-gliedrigen gesättigten Ring bilden, der noch ein zweites Heteroatom enthalten kann, mit einem Tetrahalogenmethan der Formel III

$$CX_4 \quad (III)$$

in der X Halogen bedeutet, in Gegenwart eines Initiators und eines organischen Verdünnungs- oder Lösungsmittels bei einer Temperatur im Bereich zwischen 120 und 160 °C zu einem Iminiumsalz der Formel IV

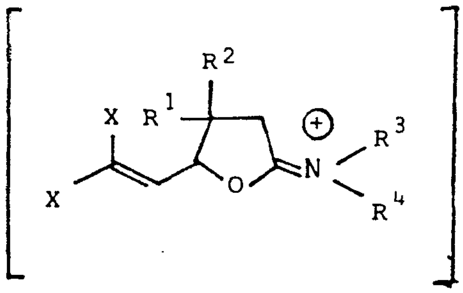

$X^{\ominus}$ (IV)

in der $R^1$, $R^2$, $R^3$, $R^4$ und X die obengenannten Bedeutungen haben, umsetzt und dieses Iminiumsalz anschließend hydrolysiert.

**Claim**

A process for the preparation of a 5-(2,2-dihalovinyl)-4,4-dialkyl-tetrahydrofuran-2-one of the formula I

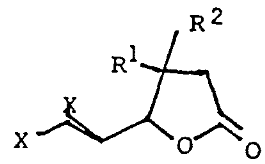

(I)

where $R^1$ and $R^2$ are each alkyl of 1 to 4 carbon atoms and X is halogen, wherein a carboxylic acid amide of the formula II

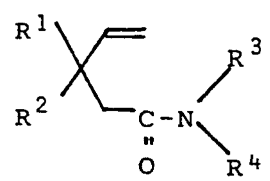

(II)

where $R^1$ and $R^2$ are each alkyl of 1 to 4 carbon atoms and $R^3$ and $R^4$ are each alkyl of 1 to 4 carbon atoms, aralkyl of 7 to 9 carbon atoms or aryl of 6 to 10 carbon atoms or together with the nitrogen on which they are present as substituents form a 5-membered or 6-membered saturated ring which may contain a further hetero-atom, is reacted with a carbon tetrahalide of the formula III

$$CX_4 \qquad (III)$$

where X is halogen, in the presence of an initiator and of an organic diluent or solvent, at from 120 to 160 °C, to give an iminium salt of the formula IV

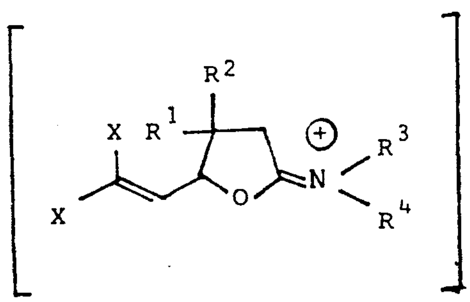

$X^{\ominus}$ (IV)

where $R^1$, $R^2$, $R^3$, $R^4$ and X have the above meanings, and this iminium salt is subsequently hydrolyzed.

**Revendication**

Procédé de préparation de 5-(2,2-dihalogenovinyl)-4,4-dialkyl-tétrahydro-furan-2-ones de formule I

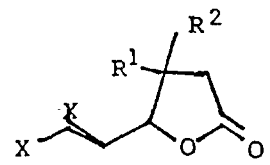

(I)

dans laquelle $R^1$ et $R^2$ représentent chacun un reste alkyle à 1 à 4 atomes de carbone et X un halogène, caractérisé par le fait qu'à une température comprise entre 120 et 160 °C, en présence d'un initiateur et d'un diluant ou solvant organique, on fait réagir un amide d'acide carboxylique de formule II

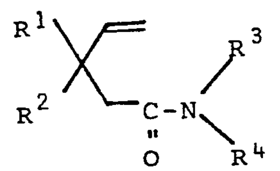

(II)

dans laquelle $R^1$ et $R^2$ représentent chacun un reste alkyle à 1 à 4 atomes de carbone et $R^3$ et $R^4$ représentent chacun un reste alkyle à 1 à 4 atomes de carbone, un reste aralkyle à 7 à 9 atomes de carbone ou un reste aryle à 6 à 10 atomes de carbone ou bien ensemble avec l'atome d'azote, dont ils sont les substituants, un noyau saturé à 5 ou 6 chaînons, qui peut encore contenir un second hétéroatome, avec un tétrahalogénométhane de formule III

$$CX_4 \quad \text{(III)}$$

où X représente un halogène, pour obtenir un sel d'iminium de formule IV

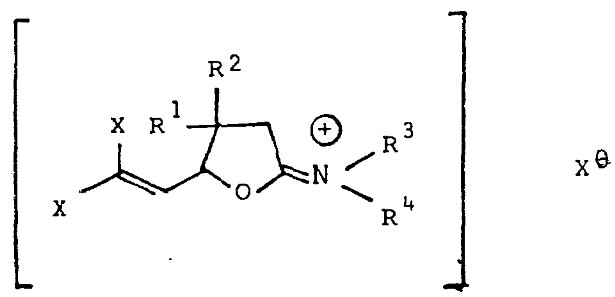

(IV)

dans laquelle $R^1$, $R^2$, $R^3$, $R^4$ et X ont les signification sus-mentionnées, et on hydrolyse ensuite ce sel d'iminium.